# EUROPÄISCHE PATENTSCHRIFT

(11) **EP 3 533 783 B1**
(45) Veröffentlichungstag und Bekanntmachung des Hinweises auf die Patenterteilung: **27.05.2020**
(21) Anmeldenummer: 18159134.8
(22) Anmeldetag: 28.02.2018
(51) Int. Cl.: C07C 253/30, C07C 255/14

(54) **VERBESSERTES VERFAHREN ZUR HERSTELLUNG VON 2-CYANOETHYLACETOACETAT**
IMPROVED PROCESS FOR THE MANUFACTURE OF 2-CYANOETHYL ACETOACETATE
PROCÉDÉ AMÉLIORÉ POUR LA PRÉPARATION DE L'ACETOACETATE DE 2-CYANOÈTHYLE

(43) Veröffentlichungstag der Anmeldung: 04.09.2019
(73) Patentinhaber: Evonik Operations GmbH, 45128 Essen (DE)
(72) Erfinder: GÄRTNER, Felix, 45721 Haltern am See (DE); LUDWIG, Bettina, 45663 Recklinghausen (DE); WILLY, Benjamin, 40211 Düsseldorf (DE)
(74) Vertreter: Evonik Patent Association

(56) Entgegenhaltungen:
- LARS B?RFACKER ET AL: "Discovery of BAY 94-8862: A Nonsteroidal Antagonist of the Mineralocorticoid Receptor for the Treatment of Cardiorenal Diseases", CHEMMEDCHEM, Bd. 7, Nr. 8, 12. August 2012 (2012-08-12) , Seiten 1385-1403, XP55213619, ISSN: 1860-7179, DOI: 10.1002/cmdc.201200081
- TORRES SUSANA Y ET AL: "Chemoenzymatic preparation of optically active 4-aryl-5-carboxy-6-methyl-3,4-dihydro-2(1H )-pyridone derivatives", TETRAHEDRON, Bd. 70, Nr. 31, 1. Januar 2014 (2014-01-01), Seiten 4675-4684, XP028848184, ISSN: 0040-4020, DOI: 10.1016/J.TET.2014.05.012

## Beschreibung

Die vorliegende Erfindung betrifft ein verbessertes Verfahren zur Herstellung von 2-Cyanoethylacetoacetat aus Trimethyldioxin-4-on und 2-Cyanoethanol. Die Verbesserung ergibt sich aus der Steuerung der Verhältnisse der Ausgangsmaterialien Trimethyldioxin-4-on und 2-Cyanoethanol in der Reaktionsmischung.

### Hintergrund der Erfindung

2-Cyanoethylacetoacetat (CAS-Nummer: 65193-87-5; im Folgenden "CE-AcAc") ist eine industriell wichtige Chemikalie und wird beispielsweise als Rohstoff in der Synthese von biologisch aktiven Substanzen eingesetzt.
In der Literatur sind verschiedene Synthesen für CE-AcAc beschrieben.
Darin wird CE-AcAc ("**3**") in einer Reaktionsstufe aus den Edukten Trimethyldioxin-4-on (2,2,6-Trimethyl-4*H*-1,3-Dioxin-4-on; CAS-Nummer: 5394-63-8; im Folgenden "TMD"; "**1**") und 2-Cyanoethanol (CAS-Nummer: 109-78-4; im Folgenden "CE"; "**2**") gewonnen.

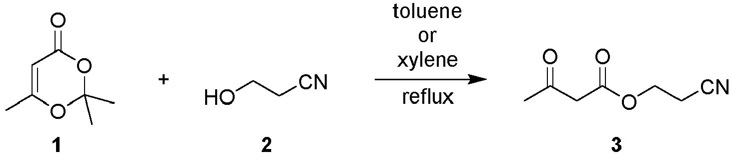

Dieses Herstellungsverfahren ist in der Literatur an zwei Stellen beschrieben, nämlich von S.Y. Torres, E. Ochoa, Y. Verdecia, F. Rebolledo, Tetrahedron 2014, 70, 4675 - 4684 (im Folgenden "Torres *et al.",* siehe Herstellung der Verbindung **7a** in Abschnitt 4.2 auf Seite 4678) und L. Bärfacker, A. Kuhl, A. Hillisch, R. Grosser, S. Figueroa-Perez, H. Heckroth, A. Nitsche, J.-K. Ergüden, H. Gielen-Haertwig, K.-H. Schlemmer, J. Mittendorf, H. Paulsen, J. Platzek, P. Kolkhof, ChemMedChem 2012, 7, 1385-1403 (im Folgenden "Bärfacker et al.; siehe Herstellung der Verbindung 26b auf Seite 1394).

Die Verfahren dieser beiden Literaturstellen zeichnen sich dadurch aus, dass die beiden Edukte TMD und CE äquimolar (Bärfacker *et al.)* bzw. mit einem leichten Überschuss an CE (Torres *et al.)* in einem Reaktor vorgelegt werden und die Mischung erhitzt wird.

Die beiden Verfahren des Standes der Technik stellen damit eine zuverlässige Möglichkeit dar, CE-AcAc herzustellen, und liefern dieses in guter Ausbeute. Für das von Bärfacker *et al.* beschriebene Verfahren wird eine Ausbeute von 88 %, für das von Torres *et al.* eine Rohausbeute von 95 % beschrieben. Erfahrungsgemäß ist die Reinheit des Produkts bei dieser Synthesemethode < 90%. Gerade für großtechnische Anwendungen besteht aber das Bedürfnis, die Ausbeute an CE-AcAc sowie die Raum-Zeit-Ausbeute in diesem Verfahren weiter zu steigern. Zusätzlich ist es wünschenswert, ein möglichst reines Produkt zu erhalten.

Die Aufgabe der vorliegenden Erfindung bestand deshalb darin, ein Verfahren zur Herstellung von CE-AcAc zur Verfügung zu stellen, welches eine gegenüber den Verfahren des Standes der Technik verbesserte Ausbeute und/ oder ein Produkt mit verbesserter Reinheit aufweist.

Es wurde nun überraschend ein Verfahren gefunden, welches diese Aufgabe löst.

### Detaillierte Beschreibung der Erfindung

Die Erfindung ist damit ein Verfahren zur Herstellung von CE-AcAc umfassend die Umsetzung von CE mit TMD zu CE-AcAc in einer Reaktionsmischung **R,**
dadurch gekennzeichnet, dass CE in der Reaktionsmischung **R** während der Umsetzung im Unterschuss von ≤ 80 Mol.-%, bezogen auf die Menge von TMD in der Reaktionsmischung **R,** vorliegt.

Es wurde nämlich überraschend festgestellt, dass sich durch die Einhaltung dieses Unterschusses in der Reaktionsmischung **R** eine deutliche Verbesserung gegenüber dem Stand der Technik, in dem CE und TMD äquimolar (Bärfacker *et al.)* bzw. mit einem leichten Überschuss an CE (Torres *et al.)* vorgemischt und umgesetzt werden, ergibt. Wie in den nachstehenden Beispielen gezeigt, zeigt sich dies in einer deutlichen Verbesserung an Ausbeute und Reinheit des Produktes CE-AcAc. Weitere Verbesserungen bei noch niedrigeren Unterschussverhältnissen wurden beobachtet. Bevorzugter sind deshalb die Fälle, in denen der Unterschuss von CE in der Reaktionsmischung **R,** bezogen auf die Menge von TMD in der Reaktionsmischung **R,** ≤ 76 Mol.-%, bevorzugter ≤ 60 Mol.-%, bevorzugter ≤ 57 Mol.-%, bevorzugter ≤ 50 Mol.-%, bevorzugter ≤ 47.5 Mol.-%, noch bevorzugter ≤ 40 Mol.-%, noch bevorzugter ≤ 38 Mol.-%, noch mehr bevorzugter ≤ 30 Mol.-%, noch mehr bevorzugter ≤ 28.5 Mol.-%, noch mehr bevorzugter ≤ 20 Mol.-%, noch mehr bevorzugter ≤ 19 Mol.-%, noch mehr bevorzugter ≤ 10 Mol.-%, noch mehr bevorzugter ≤ 9.5 Mol.-%, noch mehr bevorzugter ≤ 5 Mol.-%, noch mehr bevorzugter ≤ 1 Mol.-% beträgt.

Eine deutliche Verbesserung in Ausbeute und Reinheit wurde im Vergleich zum äquimolaren Einsatz von CE und TMD bzw. dem Einsatz eines leichten Unterschusses von 0.95 molaren Äquivalenten von CE bezogen auf TMD in den Fällen beobachtet, in denen der Unterschuss von CE in der Reaktionsmischung **R** bezogen auf die Menge von TMD in der Reaktionsmischung **R** ≤ 47.5 Mol.-% beträgt. Dieser Fall ist deshalb besonders vorteilhaft und überraschend.

Es versteht sich von selbst, dass, auch wenn der Wert "≤ 80 Mol.-%" rein mathematisch den Wert "0" umfasst, im Sinne der Erfindung jedoch damit gemeint ist, dass stets CE zumindest zu einem kleinen Anteil in der Reaktionsmischung **R** enthalten sein muss, zum Beispiel in einem Gehalt von ≥ 0.001 mol-%. Das Entsprechende gilt für die anderen angegebenen Zahlenwerte.

Demnach kann im erfindungsgemäßen Verfahrens der Unterschuss von CE in der Reaktionsmischung **R** bezogen auf die Menge von TMD in der Reaktionsmischung **R** auch insbesondere 0.001 - 80 Mol.-%, bevorzugt 0.001 - 76 Mol.-%, bevorzugter 0.001 - 60 Mol-%, noch bevorzugter 0.001 - 57 Mol.-%, noch mehr bevorzugter 0.001 - 50 Mol.-%, noch mehr bevorzugter 0.001 - 47.5 Mol.-%, noch mehr bevorzugter 0.001 - 40 Mol-%, noch mehr bevorzugter 0.001 - 38 Mol.-%, noch mehr bevorzugter 0.001 - 30 Mol.-%, noch mehr bevorzugter 0.001 - 28.5 Mol.-%, noch bevorzugter 0.001 - 20 Mol.-%, noch mehr bevorzugter 0.001 - 19 Mol.-%, noch mehr bevorzugter 0.001 - 10 Mol.-%, noch mehr bevorzugter 0.001 - 9.5 Mol.-%, noch mehr bevorzugter 0.001 - 5 Mol-%, noch mehr bevorzugter 0.001 - 1 Mol.-% betragen.

Um die Vorteile der vorliegenden Erfindung zu erhalten, ist es nicht nötig, dass der Unterschuss von CE gegenüber TMD während der kompletten Reaktionszeit, das heißt von Anfang bis Ende der Umsetzung von CE und TMD zu CE-AcAc, vorliegt. Es ist nämlich so, dass jede Phase während der Reaktion, in der CE im erfindungsgemäßen Unterschuss zu TMD vorliegt, die Vorteile der Erfindung, also eine höhere Ausbeute und Reinheit, verglichen mit dem Fall, in dem in der gegebenen Phase dieser Unterschuss nicht gegeben war, erbringt.

Im Lichte der möglichst ökonomischen Umsetzung und der größtmöglichen Ausnutzung der Vorteile der Erfindung ist es jedoch von Vorteil, den Zeitraum, in dem der erfindungsgemäße Unterschuss von CE gegenüber TMD vorliegt, möglichst auszudehnen, idealerweise über die gesamte Reaktionszeit der Umsetzung von CE und TMD zu CE-AcAc.

Vorteilhaft ist es deshalb, wenn der Unterschuss von ≤ 80 Mol.-% von CE in der Reaktionsmischung **R** bezogen auf die Menge von TMD in der Reaktionsmischung **R** so lange vorliegt, wie mindestens 1 Mol.-%, bevorzugt mindestens 10 Mol.-%, noch bevorzugter mindestens 20 Mol.-%, noch bevorzugter mindestens 30 Mol.-%, noch bevorzugter mindestens 40 Mol.-%, noch bevorzugter mindestens 50 Mol.-%, noch bevorzugter mindestens 60 Mol.-%, noch bevorzugter mindestens 70 Mol.-%, noch bevorzugter mindestens 80 Mol.-%, noch bevorzugter mindestens 90 Mol.-%, noch bevorzugter mindestens 95 Mol.-%, noch bevorzugter mindestens 99 Mol.-% der im Verfahren eingesetzten Gesamtmenge an CE mit TMD zu CE-AcAc reagieren. Noch bevorzugter liegt der Unterschuss von ≤ 80 Mol.-% von CE in der Reaktionsmischung **R** bezogen auf TMD in der Reaktionsmischung **R** über die gesamte Reaktionszeit der Umsetzung des CE mit TMD zu CE-AcAc vor.

Noch vorteilhafter ist es, wenn der Unterschuss von ≤ 50 Mol.-% von CE in der Reaktionsmischung **R** bezogen auf TMD in der Reaktionsmischung **R** so lange vorliegt, wie mindestens 1 Mol.-%, bevorzugt mindestens 10 Mol.-%, noch bevorzugter mindestens 20 Mol.-%, noch bevorzugter mindestens 30 Mol.-%, noch bevorzugter mindestens 40 Mol.-%, noch bevorzugter mindestens 50 Mol.-%, noch bevorzugter mindestens 60 Mol.-%, noch bevorzugter mindestens 70 Mol.-%, noch bevorzugter mindestens 80 Mol.-%, noch bevorzugter mindestens 90 Mol.-%, noch bevorzugter mindestens 95 Mol.-%, noch bevorzugter mindestens 99 Mol.-% der im Verfahren eingesetzten Gesamtmenge an CE mit TMD zu CE-AcAc reagieren. Noch bevorzugter liegt der Unterschuss von ≤ 50 Mol.-% von CE in der Reaktionsmischung **R** bezogen auf TMD in der Reaktionsmischung **R** über die gesamte Reaktionszeit der Umsetzung des CE mit TMD zu CE-AcAc vor.

Noch vorteilhafter ist es, wenn der Unterschuss von ≤ 47.5 Mol.-% von CE in der Reaktionsmischung **R** bezogen auf TMD in der Reaktionsmischung **R** so lange vorliegt, wie mindestens 1 Mol.-%, bevorzugt mindestens 10 Mol.-%, noch bevorzugter mindestens 20 Mol.-%, noch bevorzugter mindestens 30 Mol.-%, noch bevorzugter mindestens 40 Mol.-%, noch bevorzugter mindestens 50 Mol.-%, noch bevorzugter mindestens 60 Mol.-%, noch bevorzugter mindestens 70 Mol.-%, noch bevorzugter mindestens 80 Mol.-%, noch bevorzugter mindestens 90 Mol.-%, noch bevorzugter mindestens 95 Mol.-%, noch bevorzugter mindestens 99 Mol.-% der im Verfahren eingesetzten Gesamtmenge an CE mit TMD zu CE-AcAc reagieren. Noch bevorzugter liegt der Unterschuss von ≤ 47.5 Mol.-% von CE in der Reaktionsmischung **R** bezogen auf TMD in der Reaktionsmischung **R** über die gesamte Reaktionszeit der Umsetzung des CE mit TMD zu CE-AcAc vor.

Der Umsatz von CE und TMD zu CE-AcAc in der Reaktionsmischung **R** kann vom Fachmann routinemäßig bestimmt werden.
So kann das Auftreten des Produktes CE-AcAc sowie die Abnahme der Edukte CE und TMD in der Reaktionsmischung **R** gaschromatographisch verfolgt werden.
In der Ausführungsform, in dem das Verfahren in einem Reaktionsgefäß mit angeschlossener Destillationskolonne stattfindet, kann der Beginn des Umsatzes von CE und TMD zum Produkt CE-AcAc außerdem durch die Kondensierung der ersten Tropfen an Aceton in der Destillationskolonne bestimmt werden.

Dabei kann in den Fällen, in denen der erfindungsgemäße Unterschuss nicht über die gesamte Reaktionszeit vorliegt (also zum Beispiel nur so lange, wie 1 Mol.-%, 10 Mol.-%, 20 Mol.-% etc. der im Verfahren eingesetzten Menge von CE mit TMD reagieren), der Unterschuss zu jeder Zeit während der Umsetzung vorliegen, zum Beispiel zum Beginn der Umsetzung, zum Ende der Umsetzung oder auch während der Umsetzung.

Beispielsweise kann in einer Ausführungsform der Erfindung das Verfahren so ablaufen, dass in der Reaktionsmischung **R** zunächst ein Überschuss von CE zu TMD vorliegt, und sobald 20 Mol.-% des ursprünglich in der Reaktionsmischung **R** eingesetzten CEs mit TMD zu CE-AcAc reagiert haben, ein Unterschuss von ≤ 80 Mol.-%, bevorzugt≤ 47.5 Mol.-% von CE bezogen auf TMD in der Reaktionsmischung **R** durch Zugabe von TMD oder einer Mischung von TMD und CE, in welcher CE im Unterschuss von < 80 Mol.-%, bevorzugt≤ 47.5 Mol.-%, zu TMD vorliegt, zur Reaktionsmischung **R** eingestellt wird.

In einer anderen Ausführungsform der Erfindung kann das Verfahren so ablaufen, dass in der Reaktionsmischung **R** zunächst ein Unterschuss von ≤ 80 Mol.-%, bevorzugt≤ 47.5 Mol.-%, von CE zu TMD vorliegt, und sobald ein bestimmter Anteil, zum Beispiel 80 Mol.-% des ursprünglich in der Reaktionsmischung **R** eingesetzten CEs mit TMD zu CE-AcAc reagiert haben, ein Überschuss durch Zugabe von weiterem CE oder einer Mischung von CE und TMD, in welcher CE im Überschuss von > 80 Mol.-% zu TMD vorliegt, zur Reaktionsmischung **R** eingestellt wird.

In einer weiteren und bevorzugten Ausführungsform liegt im erfindungsgemäßen Verfahren zu Beginn der Umsetzung ein Unterschuss von ≤ 80 Mol.-%, bevorzugt ≤ 76 Mol.-%, bevorzugter ≤ 60 Mol.-%, noch bevorzugter ≤ 57 Mol.-%, noch mehr bevorzugter ≤ 50 Mol.-%, noch mehr bevorzugter ≤ 47.5 Mol.-%, noch mehr bevorzugter ≤ 40 Mol.-%, noch mehr bevorzugter ≤ 38 Mol.-%, noch mehr bevorzugter ≤ 30 Mol.-%, noch mehr bevorzugter ≤ 28.5 Mol.-%, noch mehr bevorzugter ≤ 20 Mol.-%, noch mehr bevorzugter ≤ 19 Mol.-%, noch mehr bevorzugter ≤ 10 Mol.-%, noch mehr bevorzugter ≤ 9.5 Mol.-%, noch mehr bevorzugter ≤ 5 Mol.-%, noch mehr bevorzugter ≤ 1 Mol.-% an CE in der Reaktionsmischung **R** bezogen auf die Menge von TMD in der Reaktionsmischung **R** vor, und dieser Unterschuss liegt vor, bis mindestens 1 Mol.-%, bevorzugt mindestens 10 Mol.-%, noch bevorzugter mindestens 20 Mol.-%, noch bevorzugter mindestens 30 Mol.-%, noch bevorzugter mindestens 40 Mol.-%, noch bevorzugter mindestens 50 Mol.-%, noch bevorzugter mindestens 60 Mol.-%, noch bevorzugter mindestens 70 Mol.-%, noch bevorzugter mindestens 80 Mol.-%, noch bevorzugter mindestens 90 Mol.-%, noch bevorzugter mindestens 95 Mol.-%, noch bevorzugter mindestens 99 Mol.-% der im Verfahren eingesetzten Menge an CE mit TMD zu CE-AcAc reagiert haben, wobei danach dann in der Reaktionsmischung **R** ein Verhältnis von > 80 Mol.-% an CE zu TMD, bevorzugt ein äquimolares Verhältnis oder ein molarer Überschuss an CE zu TMD eingestellt wird.

Diese Umsetzung wird ansonsten gemäß den dem Fachmann bekannten Bedingungen, die auch in den Literaturstellen von Bärfacker *et al.* oder Torres *et al.* beschrieben sind, vorgenommen.

Wie sich aus der Reaktionsgleichung ergibt, entsteht bei der Umsetzung von TMD und CE zu CE-AcAc immer zu einem gewissen Anteil Aceton als Nebenprodukt. In der Reaktionsmischung **R** wird Aceton deshalb stets zu einem gewissen geringen Anteil (ca. mindestens 0.5 bis mindestens 10 Gew.-% bezogen auf das Gesamtgewicht die Reaktionsmischung **R**) vorliegen.

Die Reaktionsmischung **R** umfasst daneben in einer Ausführungsform ein von Aceton verschiedenes Lösungsmittel ausgewählt aus von Aceton verschiedenen organischen Lösungsmitteln, Wasser.

Solche organischen Lösungsmittel sind insbesondere ausgewählt aus der Gruppe bestehend aus aromatischen Lösungsmitteln wie bevorzugt Toluol, Benzol, Xylol, bevorzugter Xylol, noch bevorzugter o-Xylol.

Bevorzugt umfasst die Reaktionsmischung kein von Aceton verschiedenes Lösungsmittel. "Kein von Aceton verschiedenes Lösungsmittel" bedeutet erfindungsgemäß insbesondere, dass der Gesamtanteil von Wasser und jeglicher von Aceton, TMD, CE und CE-AcAc verschiedenen organischen Substanz < 2 Gew.-%, bevorzugter < 1 Gew.-%, noch bevorzugter < 0.1 Gew.-%, bezogen auf das Gesamtgewicht der Reaktionsmischung **R,** ist.

"Gegebenenfalls ein von Aceton verschiedenes Lösungsmittel" bedeutet erfindungsgemäß, dass ein von Aceton verschiedenes Lösungsmittel umfasst ist oder nicht, bevorzugt kein von Aceton verschiedenes Lösungsmittel umfasst ist.

Im erfindungsgemäßen Verfahren können CE, TMD und gegebenenfalls das von Aceton verschiedene Lösungsmittel in jedem dem Fachmann bekannten Reaktionsgefäß vorgelegt werden. Solche Reaktionsgefäße können kontinuierlich oder batchweise betrieben werden. In einer Ausführungsform kann die Mischung in einem Reaktionsgefäß mit angeschlossener Destillationskolonne umgesetzt werden. Diese ermöglicht es, das als Produkt aus der Umsetzung entstehende Aceton abzudestillieren.

Die Temperatur, bei der CE mit TMD in der Reaktionsmischung **R** umgesetzt wird, ist nicht besonders beschränkt. Typischerweise liegt sie im Bereich von 50 °C bis 150 °C, bevorzugt 60 °C bis 140 °C, noch bevorzugter 70 °C bis 134 °C, noch bevorzugter 80 °C bis 130 °C, noch bevorzugter 90 °C bis 120 °C, noch bevorzugter 105 °C bis 110 °C.

Der Druck beträgt im erfindungsgemäßen Verfahren bevorzugt 0.5 bis 1.3 bar, bevorzugter Normaldruck (1 bar).

Im Sinne des möglichst sparsamen Einsatzes der Ausgangsstoffe ist es im erfindungsgemäßen Verfahren bevorzugt, wenn die Gesamtmenge an im Verfahren eingesetzten CE 50 bis 150 Mol.-%, noch bevorzugter 90 bis 120 Mol.-%, noch mehr bevorzugter 90 bis 110 Mol.-%,noch viel mehr bevorzugter 90 bis 105 Mol.-%, und am bevorzugtesten 98 bis 99 Mol.-%, jeweils bezogen auf die Gesamtmenge an im Verfahren eingesetzten TMD, beträgt.

Das Verfahren kann demnach über verschiedene Ausführungsformen verwirklicht werden.

In einer weiteren bevorzugten Ausführungsform 1) umfasst das erfindungsgemäße Verfahren die folgenden Schritte (a) bis (c):
(a) CE und TMD werden im molaren Verhältnis CE : TMD = ≤ 0.8 : 1, bevorzugter ≤ 0.76 : 1, bevorzugter ≤ 0.6 : 1, bevorzugter ≤ 0.57 : 1, bevorzugter ≤ 0.5 : 1, bevorzugter ≤ 0.475 : 1, bevorzugter ≤ 0.4 : 1, bevorzugter ≤ 0.38 : 1, bevorzugter ≤ 0.3 : 1, bevorzugter ≤ 0.285 : 1, bevorzugter ≤ 0.2 : 1, bevorzugter ≤ 0.19 : 1, bevorzugter ≤ 0.1 : 1, bevorzugter ≤ 0.095 : 1, bevorzugter ≤ 0.05 : 1, bevorzugter ≤ 0.01 : 1, gegebenenfalls mit einem von Aceton verschiedenen Lösungsmittel, bei einer Temperatur von 50 °C bis 150 °C, bevorzugt 60 °C bis 140 °C, noch bevorzugter 70 °C bis 134 °C, noch bevorzugter 80 °C bis 130 °C, noch bevorzugter 90 °C bis 120 °C, noch bevorzugter 105 °C bis 110 °C gemischt
   oder
   CE und TMD werden, gegebenenfalls mit einem von Aceton verschiedenen Lösungsmittel, bei einer Temperatur < 50 °C und im molaren Verhältnis CE : TMD = ≤ 0.8 : 1, bevorzugter ≤ 0.76 : 1, bevorzugter ≤ 0.6 : 1, bevorzugter ≤ 0.57 : 1, bevorzugter ≤ 0.5 : 1, bevorzugter ≤ 0.475 : 1, bevorzugter ≤ 0.4 : 1, bevorzugter ≤ 0.38 : 1, bevorzugter ≤ 0.3 : 1, bevorzugter ≤ 0.285 : 1, bevorzugter ≤ 0.2 : 1, bevorzugter ≤ 0.19 : 1, bevorzugter ≤ 0.1 : 1, bevorzugter ≤ 0.095 : 1, bevorzugter ≤ 0.05 : 1, bevorzugter ≤ 0.01 : 1 gemischt und die resultierende Mischung auf eine Temperatur von 50 °C bis 150 °C, bevorzugt 60 °C bis 140 °C, noch bevorzugter 70 °C bis 134 °C, noch bevorzugter 80 °C bis 130 °C, noch bevorzugter 90 °C bis 120 °C, noch bevorzugter 105 °C bis 110 °C erhitzt,
   wodurch eine Reaktionsmischung **R** umfassend CE und TMD, wobei CE in der Reaktionsmischung **R** im Unterschuss von von ≤ 80 Mol.-%, bevorzugt ≤ 76 Mol.-%, bevorzugter ≤ 60 Mol.-%, noch bevorzugter ≤ 57 Mol.-%, noch mehr bevorzugter ≤ 50 Mol.-%, noch mehr bevorzugter ≤ 47.5 Mol.-%, noch mehr bevorzugter ≤ 40 Mol.-%, noch mehr bevorzugter ≤ 38 Mol.-%, noch mehr bevorzugter ≤ 30 Mol.-%, noch mehr bevorzugter ≤ 28.5 Mol.-%, noch mehr bevorzugter ≤ 20 Mol.-%, noch mehr bevorzugter ≤ 19 Mol.-%, noch mehr bevorzugter ≤ 10 Mol.-%, noch mehr bevorzugter ≤ 9.5 Mol.-%, noch mehr bevorzugter ≤ 5 Mol.-%, noch mehr bevorzugter ≤ 1 Mol.-%, bezogen auf die Menge von TMD in der Reaktionsmischung **R,** vorliegt, erhalten wird;
(b) CE in der Reaktionsmischung **R** wird teilweise oder vollständig mit TMD in der Reaktionsmischung zu CE-AcAc umgesetzt;
(c) während oder nach Schritt (b), bevorzugt nach Schritt (b), wird weiteres CE zur Reaktionsmischung **R** gegeben, wobei das CE gegebenenfalls in einer Mischung mit TMD zugegeben wird.

Bevorzugt wird in Schritt (c) CE so zugegeben, dass der Unterschuss von CE in der Reaktionsmischung **R** gegenüber TMD in der Reaktionsmischung **R** so lange vorliegt, wie mindestens 1 Mol.-%, bevorzugt mindestens 10 Mol.-%, noch bevorzugter mindestens 20 Mol.-%, noch bevorzugter mindestens 30 Mol.-%, noch bevorzugter mindestens 40 Mol.-%, noch bevorzugter mindestens 50 Mol.-%, noch bevorzugter mindestens 60 Mol.-%, noch bevorzugter mindestens 70 Mol.-%, noch bevorzugter mindestens 80 Mol.-%, noch bevorzugter mindestens 90 Mol.-%, noch bevorzugter mindestens 95 Mol.-%, noch bevorzugter mindestens 99 Mol.-% der im Verfahren eingesetzten Gesamtmenge an CE mit TMD zu CE-AcAc reagieren.

In einer noch bevorzugteren Ausführungsform der vorgenannten Ausführungsform 1) wird in Schritt (c) während oder nach Schritt (b), bevorzugt nach Schritt (b), weiteres CE zur Reaktionsmischung **R** gegeben und parallel mit der Zugabe des CE wird TMD, getrennt von CE, zur Reaktionsmischung **R** zudosiert.

In einer weiteren bevorzugten Ausführungsform 2) des erfindungsgemäßen Verfahren umfasst dieses die folgenden Schritte (a) bis (c):
(a) TMD wird, gegebenenfalls in Mischung mit einem von Aceton verschiedenen Lösungsmittel, bevorzugt ausgewählt aus Toluol, Benzol, Xylol, bevorzugter Xylol, vorgelegt und auf eine Temperatur im Bereich von 50 °C bis 150 °C, bevorzugt 60 °C bis 140 °C, noch bevorzugter 70 °C bis 134 °C, noch bevorzugter 80 °C bis 130 °C, noch bevorzugter 90 °C bis 120 °C, noch bevorzugter 105 °C bis 110 °C erhitzt,
(b) CE wird, gegebenenfalls in einer Mischung mit TMD, zugegeben, so dass eine Reaktionsmischung **R** umfassend CE, TMD und gegebenenfalls ein von Aceton verschiedenes Lösungsmittel, wobei in der Reaktionsmischung **R** ein Unterschuss an CE von ≤ 80 Mol.-% bevorzugt ≤ 76 Mol.-%, bevorzugter ≤ 60 Mol.-%, noch bevorzugter ≤ 57 Mol.-%, noch mehr bevorzugter ≤ 50 Mol.-%, noch mehr bevorzugter ≤ 47.5 Mol.-%, noch mehr bevorzugter ≤ 40 Mol.-%, noch mehr bevorzugter ≤ 38 Mol. %, noch mehr bevorzugter ≤ 30 Mol.-%, noch mehr bevorzugter ≤ 28.5 Mol.-%, noch mehr bevorzugter ≤ 20 Mol.-%, noch mehr bevorzugter ≤ 19 Mol.-%, noch mehr bevorzugter ≤ 10 Mol.-%, noch mehr bevorzugter ≤ 9.5 Mol.-%, noch mehr bevorzugter ≤ 5 Mol.-%, noch mehr bevorzugter ≤ 1 Mol.-%, bezogen auf die Menge von TMD in der Reaktionsmischung **R,** vorliegt, erhalten wird,
(c) CE wird mit TMD in der Reaktionsmischung **R** zu CE-AcAc umgesetzt.

Es versteht sich von selbst, dass der Schritt (c) mindestens teilweise schon während der Zugabe von CE zur Reaktionsmischung R in Schritt (b) ablaufen kann.

In einer noch bevorzugteren Ausführungsform der vorgenannten Ausführungsform 2) wird in Schritt (b) weiteres CE zur Reaktionsmischung **R** gegeben und parallel mit der Zugabe des CE wird TMD, getrennt von CE, zur Reaktionsmischung **R** zudosiert.

Dies ermöglicht es auf besonders effiziente Art, die Konzentration von CE in der Reaktionsmischung **R** dauerhaft niedrig zu halten. Bevorzugt wird CE in so kleinen Portionen zu TMD gegeben, dass nach Zugabe einer Portion CE dieses vollständig mit TMD zu CE-AcAc reagiert, bevor die folgende Portion CE zugegeben wird. Dies erreicht man zum Beispiel durch langsames Dosieren von CE zu der Reaktionsmischung **R.**

Die vorliegende Erfindung betrifft ein Verfahren zur Umsetzung von CE und TMD. Es wurde beobachtet, dass durch die veränderte Zugabe eine verbesserte Ausbeute an CE-AcAc gegenüber den herkömmlichen Verfahren erreicht wird. Es wird erwartet, dass ähnliche Beobachtung bezüglich verbesserter Ausbeute etc. auch mit anderen CN-haltigen Alkoholen und/oder anderen von 1,3-Dioxin-4-on abgeleiteten Stoffen gemacht werden können.

Die folgenden Beispiele sollen die vorliegende Erfindung erläutern, ohne diese zu beschränken.

### Beispiele

### 1. Verwendete Chemikalien

In den folgenden Beispielen wurden jeweils die folgenden Chemikalien in folgender Menge eingesetzt:
Trimethyldioxin-4-on (TMD): 916.8 g (Reinheit 93.0%, 6.0 Mol, 917 mL);
2-Cyanoethanol (CE): 409.2 g (Reinheit 99.0%, 5.7 Mol, 386 mL, 0.95 molare Äquivalente bezogen auf TMD).

### 2. Erfinderische Beispiele E1 - E15, Vergleichsbeispiel V1

Im erfinderischen Beispiel **E1** wurden 916.8 g (Reinheit 93.0%; 6.0 Mol) TMD in einem 2-L-Doppelmantelgefäß mit Rückflusskühler, Innenthermometer, Destillationsaufsatz, Tropftrichter und KPG-Rührer bei Raumtemperatur vorgelegt. Danach wurde der Mantel des Doppelwandgefäßes auf 134 °C geheizt. Sobald eine Innentemperatur von 105 °C erreicht war, wurden 0.95 molare Äquivalente innerhalb 1 Stunde tropfenweise zugegeben. Noch während der Zugabe des CE fand die Umsetzung des CE und TMD zu CE-AcAc statt, was an der Entstehung des Acetons und dessen Reflux am Rückflusskühler ersichtlich war. Die erhaltene Reaktionsmischung wurde so lange umgesetzt, bis eine Innentemperatur von 130 °C erreicht war (ca. 30 Minuten nach Beendigung der Zugabe).
Dann wurde innerhalb von 30 - 40 min auf 80 °C abgekühlt und das restliche Aceton im Vakuum (80 °C, 5 mbar, 30 min) entfernt.

In den erfinderischen Beispielen **E2** - **E15** und im Vergleichsbeispiel **V1** wurde jeweils in einem 2-L-Doppelmantelgefäß mit Rückflusskühler, Innenthermometer, Destillationsaufsatz, Tropftrichter und KPG-Rührer 916.8 g (Reinheit 93.0%; 6.0 Mol) TMD und die in der Tabelle 1 (Spalte "*CE Menge in Vorlage in g"*) beschriebene Menge an CE (Reinheit 99.0%) bei Raumtemperatur vorgelegt. Danach wurde der Mantel des Doppelwandgefäßes auf 134 °C geheizt.

Sobald der Umsatz an CE und TMD stattfand, was an der Entstehung von Aceton am Rückflusskühler zu erkennen war, wurde das restliche CE (insgesamt 0.95 molare Äquivalente bezogen auf TMD) innerhalb von 1 h zugegeben. Die Umsetzung wurde gefahren, bis eine Innentemperatur von 130 °C erreicht war (ca. 30 min nach Beendigung der Zugabe).

Dann wurde innerhalb von 30 - 40 min auf 80 °C abgekühlt und das restliche Aceton im Vakuum (80 °C, 5 mbar, 30 min) entfernt.

Das Produkt wurde im Anschluss daran per Gaschromatographie (Proben verdünnt mit Toluol im Verhältnis 1:1, Säule HP-5, 30 m x 320 µm x 0.25 µm, FID-Detektor) analysiert.

Die folgende Tabelle 1 gibt die Ausbeute und Reinheit an CE-AcAc bezogen auf TMD wieder.

| **Beispiel** | **Äqu. CE vorgelegt bzgl. TMD** | **CE Menge in Vorlage [g]** | **Reinheit GC [%]** | **Ausbeute CE-AcAc bzgl. CE [%]** | **Ausbeute CE-AcAc bzgl. TMD [%]** |
|---|---|---|---|---|---|
| **E1** | 0.000 | 0 | 93.3 | 97.6 | 92.7 |
| **E2** | 0.095 | 40.9 | 94.9 | 99.7 | 94.7 |
| **E3** | 0.095 | 40.9 | 93.7 | 97.6 | 92.8 |
| **E4** | 0.190 | 81.9 | 93.5 | 97.8 | 92.9 |
| **E5** | 0.190 | 81.9 | 94.0 | 98.0 | 93.1 |
| **E6** | 0.285 | 122.8 | 93.3 | 97.1 | 92.2 |
| **E7** | 0.285 | 122.8 | 94.0 | 97.6 | 92.7 |
| **E8** | 0.285 | 122.8 | 93.6 | 97.1 | 92.3 |
| **E9** | 0.380 | 163.7 | 94.1 | 97.8 | 92.9 |
| **E10** | 0.380 | 163.7 | 95.8 | 98.6 | 93.9 |
| **E11** | 0.475 | 204.6 | 92.5 | 95.3 | 90.5 |
| **E12** | 0.475 | 204.6 | 93.0 | 96.1 | 91.3 |
| **E13** | 0.475 | 204.6 | 93.9 | 96.8 | 92 |
| **E14** | 0.570 | 245.5 | 88.5 | 90.6 | 86.1 |
| **E15** | 0.760 | 327.4 | 85.9 | 86.9 | 82.5 |
| **V1** | 0.950 | 409.2 | 80.7 | 81.1 | 77.0 |

Aus dem Vergleich von **V1** mit den Versuchen **E1** bis **E15** folgt, dass durch das erfindungsgemäße Vorgehen, das CE im Unterschuss von maximal 0.76 Mol-Äquivalenten mit TMD vorzulegen und die restliche Menge CE erst während der Reaktion zuzudosieren, um den Unterschuss möglichst lange zu halten, eine deutliche Verbesserung an Ausbeute und Reinheit des Produktes CE-AcAc erreicht wird. Aus dem Vergleich der Versuche **E1** bis **E13** mit **E14** und **E15** fällt außerdem auf, dass bei einer Vorlage von maximal 0.475 Mol-Äquivalenten, bezogen auf TMD, eine zusätzliche deutliche Steigerung gegenüber höheren Vorlagemengen CE zu TMD (0.76 Mol-Äquivalente in **E15,** 0.57 Mol-Äquivalente in **E14**) erreicht wird.

## Patentansprüche

1. Verfahren zur Herstellung von 2-Cyanoethylacetoacetat, umfassend die Umsetzung von 2-Cyanoethanol mit Trimethyldioxin-4-on zu 2-Cyanoethylacetoacetat in einer Reaktionsmischung **R,**
**dadurch gekennzeichnet, dass** 2-Cyanoethanol in der Reaktionsmischung **R** während der Umsetzung im Unterschuss von ≤ 80 Mol.-%, bezogen auf die Menge von Trimethyldioxin-4-on in der Reaktionsmischung **R,** vorliegt.

2. Verfahren nach Anspruch 1, wobei der Unterschuss von ≤ 80 Mol.-% von 2-Cyanoethanol in der Reaktionsmischung **R** bezogen auf die Menge von Trimethyldioxin-4-on in der Reaktionsmischung **R** so lange vorliegt, wie mindestens 1 Mol.-% der im Verfahren eingesetzten Gesamtmenge an 2-Cyanoethanol mit Trimethyldioxin-4-on zu 2-Cyanoethylacetoacetat reagieren.

3. Verfahren nach Anspruch 1 oder 2, wobei der Unterschuss von 2-Cyanoethanol in der Reaktionsmischung **R** bezogen auf die Menge von Trimethyldioxin-4-on in der Reaktionsmischung **R** ≤ 50 Mol.-% beträgt.

4. Verfahren nach einem der Ansprüche 1 bis 3, wobei die Reaktionsmischung **R** ein von Aceton verschiedenes Lösungsmittel ausgewählt aus von Aceton verschiedenen organischen Lösungsmitteln, Wasser umfasst.

5. Verfahren nach einem der Ansprüche 1 bis 3, wobei die Reaktionsmischung **R** kein von Aceton verschiedenes Lösungsmittel umfasst.

6. Verfahren nach einem der Ansprüche 1 bis 5, wobei die Gesamtmenge an im Verfahren eingesetzten 2-Cyanoethanol 50 bis 150 Mol.-%, bezogen auf die im Verfahren eingesetzte Gesamtmenge an Trimethyldioxin-4-on, beträgt.

7. Verfahren nach einem der Ansprüche 1 bis 6, wobei die Umsetzung von 2-Cyanoethanol und Trimethyldioxin-4-on in der Reaktionsmischung **R** bei einer Temperatur im Bereich von 50 °C bis 150 °C durchgeführt wird.

8. Verfahren gemäß einem der Ansprüche 1 bis 7, umfassend folgende Schritte (a) bis (c):
(a) 2-Cyanoethanol und Trimethyldioxin-4-on werden im molaren Verhältnis 2-Cyanoethanol : Trimethyldioxin-4-on = ≤ 0.8 : 1, gegebenenfalls mit einem von Aceton verschiedenen Lösungsmittel, bei einer Temperatur von 50 °C bis 150 °C, gemischt,
oder
2-Cyanoethanol und Trimethyldioxin-4-on werden, gegebenenfalls mit einem von Aceton verschiedenen Lösungsmittel, bei einer Temperatur < 50 °C und im molaren Verhältnis 2-Cyanoethanol : Trimethyldioxin-4-on = ≤ 0.8 : 1 gemischt und die resultierende Mischung auf eine Temperatur von 50 °C bis 150 °C erhitzt,
wodurch eine Reaktionsmischung **R** umfassend 2-Cyanoethanol und Trimethyldioxin-4-on, wobei 2-Cyanoethanol in der Reaktionsmischung **R** im Unterschuss von ≤ 80 Mol.-%, bezogen auf die Menge von Trimethyldioxin-4-on in der Reaktionsmischung **R,** vorliegt, erhalten wird;
(b) 2-Cyanoethanol in der Reaktionsmischung **R** wird teilweise oder vollständig mit Trimethyldioxin-4-on in der Reaktionsmischung zu 2-Cyanoethylacetoacetat umgesetzt;
(c) während oder nach Schritt (b) wird weiteres 2-Cyanoethanol zur Reaktionsmischung **R** gegeben, wobei das 2-Cyanoethanol gegebenenfalls in einer Mischung mit Trimethyldioxin-4-on zugegeben wird.

9. Verfahren gemäß einem der Ansprüche 1 bis 7 umfassend folgende Schritte (a) bis (c):
(a) Trimethyldioxin-4-on wird gegebenenfalls in Mischung mit einem von Aceton verschiedenen Lösungsmittel vorgelegt und auf eine Temperatur im Bereich von 50 °C bis 150 °C erhitzt,
(b) 2-Cyanoethanol wird, gegebenenfalls in einer Mischung mit Trimethyldioxin-4-on, zugegeben, so dass eine Reaktionsmischung **R** umfassend 2-Cyanoethanol, Trimethyldioxin-4-on und gegebenenfalls ein von Aceton verschiedenes Lösungsmittel, wobei in der Reaktionsmischung **R** ein Unterschuss an 2-Cyanoethanol von ≤ 80 Mol.-%, bezogen auf die Menge von Trimethyldioxin-4-on in der Reaktionsmischung **R,** vorliegt, erhalten wird,
(c) 2-Cyanoethanol wird mit Trimethyldioxin-4-on in der Reaktionsmischung **R** zu 2-Cyanoethylacetoacetat umgesetzt.

## Claims

1. Method for preparing 2-cyanoethyl acetoacetate, comprising the reaction of 2-cyanoethanol with trimethyldioxin-4-one to give 2-cyanoethyl acetoacetate in a reaction mixture **R,**
**characterized in that**, during the reaction, 2-cyanoethanol is present in the reaction mixture **R** in a deficiency of ≤ 80 mol% based on the amount of trimethyldioxin-4-one in the reaction mixture **R.**

2. Method according to Claim 1, wherein the deficiency of ≤ 80 mol% of 2-cyanoethanol in the reaction mixture **R** based on the amount of trimethyldioxin-4-one in the reaction mixture **R** is present as long as at least 1 mol% of the total amount of 2-cyanoethanol used in the method reacts with trimethyldioxin-4-one to give 2-cyanoethyl acetoacetate.

3. Method according to Claim 1 or 2, wherein the deficiency of 2-cyanoethanol in the reaction mixture **R** is ≤ 50 mol% based on the amount of trimethyldioxin-4-one in the reaction mixture **R.**

4. Method according to one of Claims 1 to 3, wherein the reaction mixture **R** comprises a solvent different to acetone, selected from organic solvents different to acetone, and water.

5. Method according to one of Claims 1 to 3, wherein the reaction mixture **R** comprises no solvent different to acetone.

6. Method according to one of Claims 1 to 5, wherein the total amount of 2-cyanoethanol used in the method is 50 to 150 mol% based on the total amount of trimethyldioxin-4-one used in the method.

7. Method according to one of Claims 1 to 6, wherein the reaction of 2-cyanoethanol and trimethyldioxin-4-one in the reaction mixture **R** is carried out at a temperature in the range from 50°C to 150°C.

8. Method according to one of Claims 1 to 7, comprising the following steps (a) to (c):
(a) 2-cyanoethanol and trimethyldioxin-4-one are mixed in the molar ratio of 2-cyanoethanol:trimethyldioxin-4-one = ≤ 0.8:1, optionally with a solvent different to acetone, at a temperature of 50°C to 150°C,
or
2-cyanoethanol and trimethyldioxin-4-one are mixed, optionally with a solvent different to acetone, at a temperature < 50°C and in the molar ratio of 2-cyanoethanol:trimethyldioxin-4-one = ≤ 0.8:1, and the resulting mixture is heated to a temperature of 50°C to 150°C,
as a result of which a reaction mixture **R** comprising 2-cyanoethanol and trimethyldioxin-4-one is obtained, wherein 2-cyanoethanol is present in the reaction mixture **R** in a deficiency of ≤ 80 mol%, based on the amount of trimethyldioxin-4-one in the reaction mixture **R;**
(b) 2-cyanoethanol in the reaction mixture **R** is partially or completely reacted with trimethyldioxin-4-one in the reaction mixture to give 2-cyanoethyl acetoacetate;
(c) during or after step (b), further 2-cyanoethanol is added to the reaction mixture **R,** wherein the 2-cyanoethanol is optionally added in a mixture with trimethyldioxin-4-one.

9. Method according to one of Claims 1 to 7, comprising the following steps (a) to (c):
(a) trimethyldioxin-4-one is initially charged optionally in a mixture with a solvent different to acetone, and heated to a temperature in the range from 50°C to 150°C,
(b) 2-cyanoethanol is added, optionally in a mixture with trimethyldioxin-4-one, such that a reaction mixture **R** is obtained comprising 2-cyanoethanol, trimethyldioxin-4-one and optionally a solvent different to acetone, wherein 2-cyanoethanol is present in the reaction mixture **R** in a deficiency of ≤ 80 mol%, based on the amount of trimethyldioxin-4-one in the reaction mixture **R,**
(c) 2-cyanoethanol is reacted with trimethyldioxin-4-one in the reaction mixture **R** to give 2-cyanoethyl acetoacetate.

## Revendications

1. Procédé pour la préparation de 2-cyanoéthylacétoacétate, comprenant la transformation de 2-cyanoéthanol avec de la triméthyldioxin-4-one pour donner du 2-cyanoéthylacétoacétate dans un mélange de réaction R,
**caractérisé en ce que** le 2-cyanoéthanol dans le mélange de réaction R pendant la transformation est présent en défaut ≤ 80 % en moles, par rapport à la quantité de triméthyldioxin-4-one dans le mélange de réaction R.

2. Procédé selon la revendication 1, le défaut ≤ 80 % en moles de 2-cyanoéthanol dans le mélange de réaction R par rapport à la quantité de triméthyldioxin-4-one dans le mélange de réaction R est présent tant qu'au moins 1 % en moles de la quantité totale utilisée dans le procédé de 2-cyanoéthanol réagit avec la triméthyldioxin-4-one pour donner du 2-cyanoéthylacétoacétate.

3. Procédé selon la revendication 1 ou 2, le défaut de 2-cyanoéthanol dans le mélange de réaction R par rapport à la quantité de triméthyldioxin-4-one dans le mélange de réaction R étant ≤ 50 % en moles.

4. Procédé selon l'une quelconque des revendications 1 à 3, le mélange de réaction R comprenant un solvant différent de l'acétone choisi parmi des solvants organiques différents de l'acétone, l'eau.

5. Procédé selon l'une quelconque des revendications 1 à 3, le mélange réactionnel R ne comprenant pas de solvant différent de l'acétone.

6. Procédé selon l'une quelconque des revendications 1 à 5, la quantité totale de 2-cyanoéthanol utilisée dans le procédé étant de 50 à 150 % en moles, par rapport à la quantité totale de triméthyldioxin-4-one utilisée dans le procédé.

7. Procédé selon l'une quelconque des revendications 1 à 6, la transformation de 2-cyanoéthanol et de triméthyldioxin-4-one dans le mélange réactionnel R étant mise en œuvre à une température dans la plage de 50 °C à 150 °C.

8. Procédé selon l'une quelconque des revendications 1 à 7, comprenant les étapes suivantes (a) à (c) :
(a) le 2-cyanoéthanol et la triméthyldioxin-4-one étant mélangés en un rapport molaire 2-cyanoéthanol:triméthyldioxin-4-one = ≤ 0,8:1, éventuellement avec un solvant différent de l'acétone, à une température de 50 °C à 150 °C,
ou
le 2-cyanoéthanol et la triméthyldioxin-4-one étant mélangés, éventuellement avec un solvant différent de l'acétone, à une température < 50 °C et en un rapport molaire 2-cyanoéthanol:triméthyldioxin-4-one = ≤ 0,8:1 et le mélange résultant étant chauffé à une température de 50 °C à 150 °C,
un mélange réactionnel R comprenant du 2-cyanoéthanol et de la triméthyldioxin-4-one étant obtenu, le 2-cyanoéthanol dans le mélange réactionnel R étant présent en défaut ≤ 80 % en moles, par rapport à la quantité de triméthyldioxin-4-one dans le mélange réactionnel R ;
(b) le 2-cyanoéthanol dans le mélange de réaction R étant partiellement ou totalement transformé avec la triméthyldioxin-4-one dans le mélange réactionnel pour donner le 2-cyanoéthylacétoacétate ;
(c) pendant ou après l'étape (b), du 2-cyanoéthanol supplémentaire étant ajouté au mélange réactionnel R, le 2-cyanoéthanol étant éventuellement ajouté dans un mélange avec de la triméthyldioxin-4-one.

9. Procédé selon l'une quelconque des revendications 1 à 7 comprenant les étapes suivantes (a) à (c) :
(a) la triméthyldioxin-4-one étant éventuellement mise à disposition en mélange avec un solvant différent de l'acétone et chauffée à une température dans la plage de 50 °C à 150 °C,
(b) le 2-cyanoéthanol étant ajouté, éventuellement en un mélange avec de la triméthyldioxin-4-one, de sorte qu'un mélange réactionnel R comprenant du 2-cyanoéthanol, de la triméthyldioxin-4-one et éventuellement un solvant différent de l'acétone est obtenu, un défaut de 2-cyanoéthanol ≤ 80 % en moles étant présent dans le mélange réactionnel R, par rapport à la quantité de triméthyldioxin-4-one dans le mélange réactionnel R,
(c) le 2-cyanoéthanol étant transformé avec la triméthyldioxin-4-one dans le mélange réactionnel R pour donner le 2-cyanoéthylacétoacétate.
